(19) Europäisches Patentamt
European Patent Office
Office européen des brevets

(11) Veröffentlichungsnummer: **0 001 294**
**B1**

(12) # EUROPÄISCHE PATENTSCHRIFT

(45) Veröffentlichungstag der Patentschrift: **02.09.81**

(51) Int Cl.³: **C 07 D 407/04,** A 61 K 31/35 // (C07D407/04, 309/00)

(21) Anmeldenummer. **78100991.5**

(22) Anmeldetag. **26.09.78**

(54) **Lasalocid-Derivate, deren Herstellung und Zwischenprodukte dafür sowie diese Derivate enthaltende pharmazeutische Präparate.**

(30) Priorität: **26.09.77 US 836350**
**03.07.78 US 921645**

(43) Veröffentlichungstag der Anmeldung: **04.04.79 Patentblatt 79/7**

(45) Bekanntmachung des Hinweises auf die Patenterteilung. **02.09.81 Patentblatt 81/35**

(84) Benannte Vertragsstaaten: **BE CH DE FR GB LU NL SE**

(56) Entgegenhaltungen:
**JOURNAL OF MEDICINAL CHEMISTRY, 1973 16(4) Seiten 397—403**

(73) Patentinhaber: **F. HOFFMANN-LA ROCHE & CO. Aktiengesellschaft, CH-4002 Basel (CH)**

(72) Erfinder: **Coffen, David, 270 Ridgewood Avenue, Glenridge N.J. (US)**

(74) Vertreter: **Lederer, Franz, Dr. et al, Patentanwälte Lederer, Meyer Lucile-Grahn-Strasse 22, D-8000 München 80 (DE)**

Anmerkung. Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art 99(1) Europäisches Patentübereinkommen).

BUNDESDRUCKEREI BERLIN

EP 0 001 294 B1

Lasalocid-Derivate, deren Herstellung und Zwischenprodukte dafür sowie diese Derivate enthaltende pharmazeutische Präparate

Die vorliegende Erfindung betrifft Lasalocid-Derivate der Formel

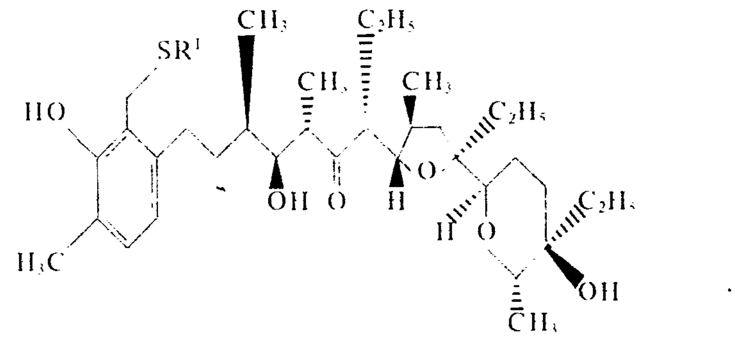

(I)

worin $R^1$ Phenyl, substituiertes Phenyl, Heteroaryl Phenyl-$C_{1-7}$-alkyl, $C_{4-7}$ Cycloalkyl, $C_{1-7}$ Alkyl, $C_{2-7}$ Alkenyl, Carbo-$C_{1-16}$alkoxy-$C_{1-7}$alkyl, Amino-$C_{1-16}$alkyl, $C_{1-7}$ Alkylamino-$C_{1-16}$alkyl, Di($C_{1-7}$alkyl)-amino-$C_{1-16}$alkyl, Hydroxy-$C_{1-16}$-alkyl, $C_{1-16}$ Alkoxy-$C_{1-16}$alkyl, $C_{1-16}$ Thioalkoxy-$C_{1-16}$alkyl, Carboxy-$C_{1-7}$alkyl oder Wasserstoff bedeutet.

Die Erfindung betrifft weiterhin ein Verfahren zur Herstellung der Verbindungen I und pharmazeutische Kompositionen auf der Basis der Verbindungen I.

Der hier verwendete Ausdruck »Alkyl« bezeichnet für sich allein oder in Verbindung mit anderen Resten eine geradkettige oder verzweigte, gesättigte Kohlenwasserstoffgruppe mit 1—16 C-Atomen. Bevorzugte Alkylgruppen sind nieder-Alkylgruppen mit 1—7 C-Atomen. Beispiele von niederen Alkylgruppen sind Methyl, Äthyl, Isopropyl und tertiär Butyl.

Beispiele von »substituiertem Phenyl« sind Alkyl-, Carboxy-, Halo-, Alkoxy- oder Nitro-substituiertes Phenyl, wie Tolyl.

Der Ausdruck »Halogen« oder »Halo« umfaßt Chlor, Brom, Fluor und Jod.

Ein Cycloalkylrest enthält 4—7 C-Atome, wie Cyclohexyl.

Der Ausdruck »Heteroaryl« bezeichnet einen unsubstituierten mono-heterocyclischen aromatischen Rest, wie Pyridyl oder Furyl.

Der Ausdruck »Phenyl-$C_{1-7}$alkyl« bezeichnet eine geradkettige oder verzweigte Alkylgruppe mit 1—7 C-Atomen, die durch eine Phenylgruppe substituiert ist, wie Benzyl.

Der Ausdruck »$C_{2-7}$ Alkenyl« bezeichnet eine geradkettige oder verzweigte Kohlenwasserstoffgruppe die eine olefinische Doppelbindung und 2—7 C-Atome aufweist.

Eine bevorzugte Gruppe von Verbindungen der Formel I sind diejenigen, in denen $R^1$ Phenyl, substituiertes Phenyl, Phenyl $C_{1-7}$alkyl, $C_{4-7}$ Cycloalkyl, $C_{1-7}$ Alkyl, Carbo-$C_{1-16}$alkoxy-$C_{1-7}$alkyl oder Wasserstoff ist. Besonders bevorzugt sind Verbindungen der Formel I, in denen $R^1$ Phenyl, Chlorphenyl, Cyclohexyl, Benzyl oder Carboxymethyl ist. Am meisten bevorzugt ist die Verbindung der Formel I, in der $R^1$ Phenyl ist, d. h. die Verbindung 1-(Phenylthiomethyl)-1-decarboxylasalocid.

Die Verbindungen der Formel I werden erfindungsgemäß dadurch erhalten, daß man eine Verbindung der Formel

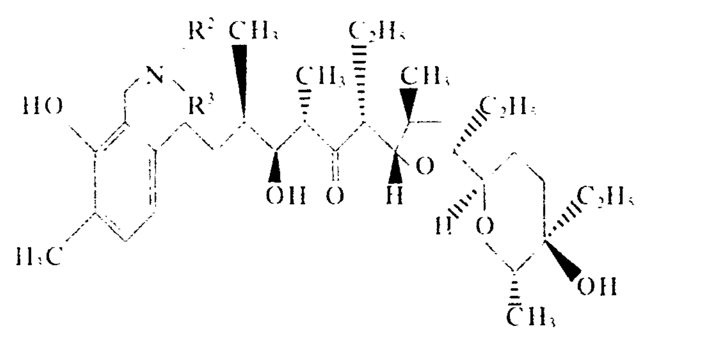

(II)

worin $R^2$ und $R^3$ $C_{1-7}$ Alkyl sind oder zusammen mit dem Stickstoffatom einen 5- oder 6gliedrigen heterocyclischen Ring, der ein weiteres Heteroatom enthalten kann, darstellen,
mit einer Verbindung der Formel $R^1SH$, worin $R^1$ die obige Bedeutung hat, in Gegenwart einer schwachen Base umsetzt.

Bei dieser Reaktion ist eine schwache Base eine solche die ausreicht, um das Orthochinon-methid und die konjugierte Base des betreffenden Mercaptans, d. h. $R^1S^{\ominus}$ zu bilden, ohne eine Retroaldolspaltung des Lasalocidmoleküls zu bewirken. Geeignete Basen sind Alkalimetall-, z. B.

Natrium- oder Kaliumcarbonate und -bicarbonate, Trialkylamine oder Pyridin. Geeignete Lösungsmittel für diese Reaktion sind Alkohole, z. B. Methanol oder Äthanol, hochsiedende Äther, Dioxan oder Tetrahydrofuran und Äthylacetat. Die Reaktionstemperatur kann zwischen Raumtemperatur und Rückflußtemperatur liegen, wobei Rückflußtemperatur des verwendeten Lösungsmittels bevorzugt ist. Bevorzugte Reaktionspartner sind Thiophenol, p-Chlorthiophenol, Cyclohexylmercaptan, Benzylmercaptan und Mercaptoessigsäure, wobei man Verbindungen der Formel I erhält, worin $R^1$ Phenyl, p-Chlorphenyl, Cyclohexyl, Benzyl bzw. Carboxymethyl ist.

Das Ausgangsmaterial der Formel II kann aus Lasalocid, einer Verbindung der Formel

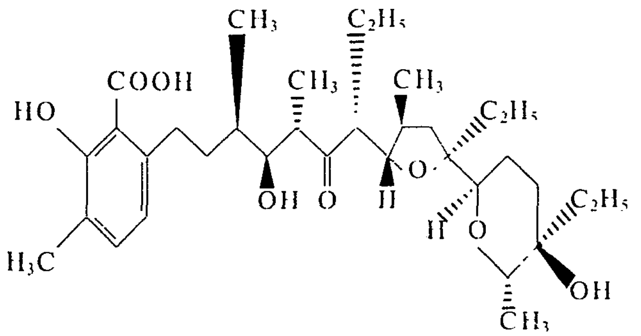

(III)

mittels einer Mannich-Reaktion, genauer mittels eines Gemisches eines Dialkylamins oder eines cyclischen Amins, wie Morpholin, Piperidin oder Pyrrolidin und p-Formaldehyd oder wäßrigem Formaldehyd erhalten werden. Geeignete Lösungsmittel für diese Reaktion sind $C_1-C_4$ Alkohole, Toluol, Benzol, chlorierte Kohlenwasserstoffe und Äther wie Dioxan oder Tetrahydrofuran. Die Reaktionstemperatur kann von 60°C bis Rückflußtemperatur des verwendeten Lösungsmittels variieren, wobei Rückflußtemperatur bevorzugt ist.

Die Verbindung der Formel III, Lasalocid, auch bekannt z. B. aus der Deutschen Offenlegungsschrift Nr. 18 01 228 als »Antibiotikum X-537 A«, ist ein kristallines Antibiotikum, das durch einen Streptomyces-Organismus produziert wird, der aus einer in Hyde Park, Massachusetts, gesammelten Bodenprobe isoliert wurde. Lyophilisierte Proben der Kultur welche die Laboratoriumsbezeichnung X-537 tragen, wurden beim United States Department of Agriculture, Agriculture Research Service, Northern Utilization Research and Development Division, Peoria, Illinois, hinterlegt. Die Kultur, die die Identifizierungsnummer NRRL 3382 durch den Agricultural Research Service erhielt, wurde durch NRRL der Öffentlichkeit zugänglich gemacht. Eine Ersatzkultur die einen nahe verwandten Stamm der ursprünglich hinterlegten Kultur NRRL 3382 darstellt, d. h sowohl gemeinsame Abstammungs- und morphologische Charakteristika aufweist, wurde beim NRRL unter der Nummer NRRL 3382R hinterlegt. Diese Kultur ist ebenso wie die ursprünglich hinterlegte Kultur NRRL 3382 öffentlich zugänglich.

Lasalocid (Antibiotikum X-537 A) wird durch Wachstum des Streptomyces-Organismus in einer belüfteten Submerskultur deren pH etwa neutral ist, d. h. etwa 6,5 bis 7,5 beträgt, hergestellt. Das Medium enthält eine Stickstoffquelle wie Hefe, ein Hefederivat, Maismehl, Bohnenmehl und ähnliches, wobei Sojabohnenmehl besonders bevorzugt ist, und eine Kohlenhydratquelle wie Zucker, Melasse und ähnliches, wobei Rohzucker besonders bevorzugt ist. Die Fermentation wird bei leicht erhöhten Temperaturen, d. h. zwischen 25°C und 35°C ausgeführt, besonders bevorzugt ist eine Inkubationstemperatur von etwa 28°C. Nach 4- bis 6tägiger Inkubation wird die Fermentationsbrühe abfiltriert und das Antibiotikum durch Extraktion erhalten.

Die Verbindungen der Formel I weisen anti-hypertensive Aktivität auf.

Unter Verwendung eines Standards als Kontrolle zu Vergleichzwecken wurde das 1-(Phenylthiomethyl)-1-decarboxylasalocid auf antihypertensive Aktivität getestet. Die Versuche wurden mit einzelnen oralen Dosen an der(DOCA-Na-Ratte) während einer Dauer von 5 Tagen durchgeführt. Nach der 5-Tage-Periode wurde das Präparat abgesetzt. Blutdruckmessungen wurden während der 5-Tage-Periode des Präparates durchgeführt, bis der Blutdruck sich auf den Ausgangswert vor der Medikation eingestellt hatte. Die tägliche Dosis betrug 10 mg/kg. Die folgenden Resultate wurden erhalten:

Orale antihypertensive Aktivität von 1-(Phenylthiomethyl)-1-decarboxylasalocid (5-tägige Verabreichung) an DOCA-Na Ratten[a])

| | Dosis (mg/kg p. o.) | N | Systolischer Blutdruck (mm Hg) | | | | | | | | | |
|---|---|---|---|---|---|---|---|---|---|---|---|---|
| | | | 1. Tag | | 2. Tag | | 3. Tag | | 4. Tag | | 5. Tag | |
| | | | vor-mittags | nach-mittags | vor-mittags | nach-mittags | vor-mittags | nach-mittags | vor-mittags | nach-mittags | vor-mittags | nach-mittags |
| Träger | – | 5 | 203 ± 2 | 194 ± 4 | 198 ± 5 | 188 ± 9 | 201 ± 4 | 195 ± 4 | 205 ± 4 | 193 ± 7 | 200 ± 4 | 188 ± 5 |
| 1-(Phenylthiomethyl)-1-decarboxylasalccid | 10.0 | 12 | 212 ± 5 | 181 ± 8 | 172 ± 6 | 160 ± 6 | 173 ± 4 | 160 ± 3 | 173 ± 5 | 151 ± 9 | 159 ± 7 | 151 ± 4 |
| | | | Herzschlag (Schläge/Min.) | | | | | | | | | |
| Träger | – | 5 | 410 ± 19 | 404 ± 9 | 406 ± 23 | 400 ± 13 | 404 ± 29 | 424 ± 24 | 442 ± 28 | 434 ± 22 | 434 ± 17 | 400 ± 20 |
| 1-(Phenylthiomethyl)-1-decarboxylasalocid | 10.0 | 12 | 428 ± 17 | 433 ± 17 | 435 ± 14 | 424 ± 19 | 414 ± 11 | 386 ± 11 | 410 ± 11 | 384 ± 12 | 403 ± 11 | 374 ± 12 |

[a]) DOCA-Na-hypertensive männliche Ratten erhielten orale Einzeldosen entweder von Träger (5% Gummi arabicum) oder 1-(Phenylthiomethyl)-1-decarboxylasalocid in Gummi arabicum an 5 aufeinanderfolgenden Tagen. Der systolische Blutdruck und der Herzschlag wurden vor der Medikation (vormittags) und 6 Stunden danach (nachmittags) gemessen. N bezeichnet die Anzahl der Versuchstiere.

1-[(Carboxymethyl)thiomethyl]-1-decarboxylasalocid, 1-(p-Chlorphenylthiomethyl)-1-decarboxylasalocid, 1-(Benzylthiomethyl)-1-decarboxylasalocid und 1-(Cyclohexylthiomethyl)-1-decarboxylasalocid wurden ebenso getestet.

Die Resultate sind in der nachstehenden Tabelle wiedergegeben. Aus den Resultaten geht hervor, daß im Einsetzen und der Dauer der antihypertensiven Aktivitäten Unterschiede existieren, daß aber die obengenannten Verbindungen eine anti-hypertensive Aktivität aufweisen, wenn man den Blutdruck am 1. Tag mit dem am 5. Tag nach Absetzen des Medikamentes vergleicht.

Wirkung auf den systoischen Blutdruck und der Herzschlag bei der wachen DOCA-Na hypertensiven Ratte*)

| Verbindung | Dosis (mg/kg p. o.) | N | Systolischer Blutdruck (mm Hg) | | | | | | | | | |
|---|---|---|---|---|---|---|---|---|---|---|---|---|
| | | | 1. Tag | | 2. Tag | | 3. Tag | | 4. Tag | | 5. Tag | |
| | | | vor-mittags | nach-mittags | vor-mittags | nnach-mittags | vor-mittags | nach-mittags | vor-mittags | nach-mittags | vor-mittags | nach-mittags |
| 1-[(Carboxymethyl-thiomethyl]-1-decarb-oxylasalocid | 10 | 6 | 226 ± 3 | 204 ± 6 | 208 ± 4 | 206 ± 6 | 207 ± 12 | 196 ± 10 | 209 ± 6 | 192 ± 7 | 211 ± 8 | 202 ± 9 |
| 1-(p-Chlorophenyl-thiomethyl)-1-decarb-oxylasalocid | 10 | 6 | 221 ± 3 | 194 ± 9 | 180 ± 11 | 171 ± 12 | 189 ± 8 | 171 ± 9 | 203 ± 11 | 187 ± 8 | 209 ± 6 | 207 ± 3 |
| 1-(Benzylthiomethyl)-1-decarboxylasalocid | 10 | 6 | 203 ± 4 | 186 ± 3 | 201 ± 2 | 181 ± 5 | 192 ± 4 | 187 ± 2 | 198 ± 4 | 203 ± 3 | 194 ± 2 | 199 ± 4 |
| 1-(Cyclohexylthio-methyl)-1-decarboxy-lasalocid | 10 | 6 | 207 ± 4 | 180 ± 7 | 201 ± 5 | 184 ± 6 | 200 ± 11 | 172 ± 7 | 196 ± 9 | 182 ± 9 | 204 ± 4 | 187 ± 6 |
| 1-[(Thiomethyl)-carb-oxymethyl]-1-decarb-oxylasalocid | 10 | 6 | 430 ± 13 | 430 ± 14 | 435 ± 14 | 448 ± 20 | 405 ± 18 | 403 ± 16 | 422 ± 24 | 417 ± 17 | 430 ± 27 | 402 ± 20 |
| 1-(p-Chlorophenylthio-methyl)-1-decarboxy-lasalocid | 10 | 6 | 435 ± 13 | 422 ± 18 | 388 ± 17 | 382 ± 16 | 400 ± 21 | 375 ± 16 | 387 ± 19 | 397 ± 20 | 418 ± 14 | 357 ± 11 |
| 1-(Benzylthiomethyl)-1-decarboxylasalocid | 10 | 6 | 455 ± 15 | 445 ± 11 | 438 ± 17 | 465 ± 15 | 425 ± 7 | 442 ± 17 | 423 ± 3 | 420 ± 14 | 435 ± 12 | 410 ± 13 |
| 1-(Cyclohexylthio-methyl)-1-decarboxy-lasalocid | 10 | 6 | 452 ± 16 | 441 ± 22 | 455 ± 16 | 407 ± 17 | 430 ± 24 | 418 ± 16 | 403 ± 15 | 415 ± 19 | 448 ± 15 | 408 ± 8 |

*) DOCA-Na-hypertensive mannliche Ratten erhielten orale Einzeldosen die der Verbindung auf 5 aufeinanderfolgenden Tagen. Der systolische Blutdruck und der Herzschlag wurden vor der Medikation (vormittags) und 6 Stunden danach (nachmittags) gemessen. N bezeichnet die Anzahl der Versuchstiere.

Beispiel A

Kapseln können folgende Bestandteile enthalten:

| | | mg/ Kapsel | mg/ Kapsel | mg/ Kapsel | mg/ Kapsel |
|---|---|---|---|---|---|
| 1. | 1-(Phenylthiomethyl)-1-decarboxylasalocid | 0,1 | 1,0 | 5,0 | 10,0 |
| 2. | Polyvinylpyrrolidon | 20,0 | 20,0 | 20,0 | 20,0 |
| 3. | Modifizierte Stärke | 55,0 | 55,0 | 55,0 | 55,0 |
| 4. | Lactose | 167,4 | 166,5 | 212,5 | 257,5 |
| 5. | Dioctylnatriumsulfosuccinat | 1,5 | 1,5 | 1,5 | 1,5 |
| 6. | Talk | 5,0 | 5,0 | 5,0 | 5,0 |
| 7. | Magnesiumstearat | 1,0 | 1,0 | 1,0 | 1,0 |
| | Füllgewicht | 250,0 | 250,0 | 300,0 | 350,0 |

Verfahren

1. Die Bestandteile 1, 3 und 5 werden gemischt.
2. Die Bestandteile 2 und 5 werden in destilliertem Wasser und/oder Alkohol gelöst, auf die richtige Konsistenz granuliert und gemahlen.
3. Trocknen im Ofen.
4. Mahlen und Mischen mit Talk und Magnesiumstearat während 2 Minuten.
5. Verkapseln.

Beispiel B

Tabletten können folgende Zusammensetzung aufweisen:

| | | mg/ Tabl. | mg/ Tabl. | mg/ Tabl. | mg/ Tabl. |
|---|---|---|---|---|---|
| 1. | 1-(Phenylthiomethyl)-1-decarboxylasalocid | 0,1 | 1 | 5 | 10 |
| 2. | Lactose | 202,9 | 202 | 232 | 261 |
| 3. | Modifizierte Stärke | 25 | 25 | 35 | 45 |
| 4. | Vorgelatinierte Stärke | 20 | 20 | 25 | 30 |
| 5. | Destilliertes Wasser q. s. | – | – | – | – |
| 6. | Magnesiumstearat | 2 | 2 | 3 | 4 |
| | Tablettengewicht | 250,0 | 250 | 300 | 350 |

Verfahren

1. Mischen der Bestandteile 1—4 in einem geeigneten Gerät.
2. Granulieren mit destilliertem Wasser auf die passende Konsistenz. Mahlen.
3. Trocknen im Ofen.
4. Mahlen und Mischen mit Magnesiumstearat während 3 Minuten.
5. Verpressen zu Tabletten.

Zur Verwendung als antihypertensive Mittel können die Verbindungen der Formel I in Form pharmazeutischer Präparate mit inerten pharmazeutischen Hilfsmitteln in für die orale Verabreichung geeignete Dosierungsformen gebracht werden. Andere Dosierungsformen, z. B. die parenterale, sind auch möglich. Beispiele von oralen Dosierungsformen sind Tabletten, Kapseln, Dragees, Suspensionen und Lösungen. Als Hilfsmittel können anorganische oder organische Stoffe wie beispielsweise Gelatine, Albumin, Lactose, Stärke, Magnesiumstearat, Konservierungsmittel (Stabilisatoren), Gleitmittel, Emulgatoren, Salze zur Veränderung des osmotischen Druckes und Puffer verwendet werden, die in solchen Präparaten üblich sind.

Es hat sich gezeigt, daß die subakute orale Verabreichung zur Behandlung des Hochdrucks bei Warmblütern, z. B. der DOCA-Na-hypertensiven Ratte, d. h. eine Dosierung bis zu 5 Tagen mit anschließendem Absetzen des Präparates am wirksamsten ist, wenn der Dosierungsbereich zwischen etwa 5 mg/kg und etwa 100 mg/kg liegt, insbesondere zwischen 5 mg/kg und 10 mg/kg. Die chronische orale Verabreichung, d. h. eine Dosierung über 5 Tage hinaus, ist am wirksamsten, wen ein niedriges Dosierungsniveau angewandt wird, d. h. niedriger als 0,1 mg/kg täglich, z. B. 0,01 mg/kg bis etwa 5 mg/kg pro Tag. Die vorstehend beschriebenen Dosierungspläne können auch bei Behandlung anderer cardiovaskulärer Symptome wie Angina pectoris, Claudicatio und verminderter Gehirndurchblutung Anwendung finden. Bei der Dosierung ist das Dosierungschema für die chronische Verabreichung besonders bevorzugt, d. h. eine Dosierung von weniger als 0,1 mg/kg pro Tag bis etwa 5 mg/kg pro Tag.

## Beispiel 1

Der Streptomycesstamm NRRL 3382 R wurde in belüfteter Submerskultur in Schüttelkolben kultiviert. Das Medium wurde durch Zusatz von Kalilauge auf pH 6,5—7,5 gebracht, darauf wurde es sterilisiert. In einem Fermentationstank wurde das Medium, das 2% Sojabohnenmehl, 2% Rohzucker, 0,5% Maisquellwasser und 0,1% $K_2HPO_4$ enthielt mit 5—10% eines Inokulums aus einer 3 Tage alten Submerskultur beimpft. Die Fermentation wurde bei 28° unter positivem Luftdruck ausgeführt wobei pro Minute und pro 150—300 Liter Flüssigkeit ein Luftstrom von 0,14—0,28 Kubikmeter aufrechterhalten wurde. Die Kultur wurde nach 4—6 Tagen Fermentation geerntet, filtriert und das Antibiotikum durch Extraktion wie folgt abgetrennt:

204 Liter Kulturlösung wurden filtriert und der feuchte Filterkuchen wurde in 100 Litern Butylacetat suspendiert und über Nacht bei Raumtemperatur gerührt. Das Gemisch wurde dann filtriert und die wäßrige Schicht abgetrennt und verworfen. Die Butylacetatlösung, die 30 Millionen Bazillus E-Einheiten aufwies, wurde unter vermindertem Druck auf 3 Liter konzentriert, mit 10%iger Natriumcarbonatlösung gewaschen und über Natriumsulfat getrocknet.

Nach weiterem Einengen auf 300 ml und Verdünnen mit 350 ml Petroläther (Sdp. 50—60°) wurden 41 g festes Material mit einem Gehalt von 25 Millionen Bazillus E-Einheiten abgetrennt. Dieser Feststoff wurde dann in einem Soxhletapparat 40 Stunden lang mit 4 Liter Petroläther (Sdp. 50—60°) extrahiert. Der Extrakt wurde unter vermindertem Druck zur Trockene gebracht, der kristalline Rückstand in Petroläther suspendiert und filtriert und lieferte 24,49 g eines Gemisches von 3-Methyl-6-[7-äthyl-4-hydroxy-3,5-dimethyl-6-oxo-7-[5-äthyl-3-methyl-5-(5-äthyl-5-hydroxy-6-methyl-2-tetrahydropyranyl)-2-tetrahydrofuryl]heptyl]salicylsäure (Lasalocid) und des Salzes davon. Die Mutterlauge lieferte weitere 5,73 g des Antibiotikums.

Nach Umkristallisieren aus Äther-Petroläther wurde das Natrium enthaltende Material in Äther gelöst und mit verdünnter Schwefelsäure gewaschen, um es in die freie Säure zu überführen. Entfernung des Äthers lieferte einen öligen Rückstand, der aus Äthanol kristallisierte und reines Lasalocid-Äthanolat lieferte. Mehrere Umkristallisationen aus Äthanol verändern den Schmelzpunkt, der bei 100—109° unscharf blieb, nicht.

## Beispiel 2

Ein Gemisch von 5 g Lasalocid-Äthanolat, 2,5 g Paraformaldehyd und 2,5 g Diäthylamin in 200 ml Toluol wurde 90 Minuten unter Rühren zum Rückfluß erhitzt. Während des Rückflußerhitzens wurde das Wasser in einer Falle gesammelt. Die erhaltene Lösung wurde mit Äther verdünnt, mit Wasser gewaschen und mit sehr verdünnter (ca. 0,05 N) Salzsäure gewaschen. Die organische Schicht wurde getrocknet, filtriert und das Lösungsmittel unter vermindertem Druck entfernt, wobei ein hellgelbes (manchmal farbloses Harz) zurückblieb. Diese rohe Mannich-Base, 1-(Diäthylaminomethyl)-1-decarboxylasalocid, enthielt Toluol, wurde aber direkt in der nächsten Stufe eingesetzt.

## Beispiel 3

Eine Lösung von 9,7 g der in Beispiel 2 hergestellten Mannich-Base, 4,5 ml Thiophenol und 4 ml Triäthylamin in 125 ml Äthanol wurde 18 Stunden zum Rückfluß erhitzt. Das Lösungsmittel wurde unter vermindertem Druck abgedampft und der Rückstand in Methylenchlorid aufgenommen. Nach

Waschen mit Wasser und verdünnter Natriumcarbonatlösung wurde die Lösung getrocknet und das Lösungsmittel abgedampft. Das Rohprodukt wurde durch Chromatographie an 200 g Silicagel gereinigt. Mit Hexan/Äthylacetat (8 : 1) wurde Thiophenol und mit Hexan/Äthylacetat (6 : 1) das Produkt, 1-(Phenylthiomethyl)-1-decarboxylasalocid, eluiert. Abdampfen des Lösungsmittels, zum Schluß unter vermindertem Druck, lieferte einen farblosen Schaum.

Beispiel 4

Eine Lösung von 1,4 g 1-(Diäthylaminomethyl)-1-decarboxylasalocid, 2 g p-Chlorthiophenol und 2 ml Triäthylamin in 50 ml Äthanol wurde 18 Stunden zum Rückfluß erhitzt. Da die Reaktion nicht vollständig war (Dünnschichtchromatogramm) wurden weitere 2 g p-Chlorthiophenol und 2 ml Triäthylamin zugesetzt und das Rückflußerhitzen für 24 Stunden fortgesetzt. Nach Abkühlen wurde das Lösungsmittel und das Amin unter vermindertem Druck abgedampft und der Rückstand in Methylenchlorid aufgenommen. Diese Lösung wurde mit wäßriger Natriumcarbonatlösung gewaschen, über Natriumsulfat getrocknet und eingedampft.

Der Rückstand war ein Gemisch, hauptsächlich aus p-Chlorthiophenol und dem gewünschten Thioäther, 1-(p-Chlorphenylthiomethyl)-1-decarboxylasalocid, das an Silicagel mit Benzoläthylacetat (2 : 1) als Elutionsmittel chromatographiert wurde. Die das Produkt enthaltenden Fraktionen (Dünnschichtchromatographie) wurden vereinigt und lieferten beim Eindampfen einen farblos festen Schaum, der bei 42—46° flüssig wurde.

Beispiel 5

1,4 g 1-(Diäthylaminomethyl)-1-decarboxylasalocid, 2 ml Cyclohexylmercaptan und 2 ml Triäthylamin wurden in 50 ml Äthanol zum Rückfluß erhitzt. Mittels des im Beispiel 4 beschriebenen Verfahrens, einschließlich des Zusatzes von weiterem Cyclohexylmercaptan und Triäthylamin nach 18 Stunden, wurde das Produkt, 1-(Cyclohexylthiomethyl)-1-decarboxylasalocid, als farbloser fester Schaum nach Reinigung an einer Silicagelkolonne erhalten.

Beispiel 6

4,5 g 1-(Diäthylaminomethyl)-1-decarboxylasalocid, 5 ml Benzylmercaptan und 5 ml Triäthylamin wurden in 100 ml Äthanol über Nacht zum Rückfluß erhitzt. Nach Kühlen und Abdampfen des Lösungsmittels unter vermindertem Druck wurde der Rückstand an Silicagel mit Hexan/Äthylacetat (5 : 1) chromatographiert. Die das Produkt erhaltenden Fraktionen wurden vereinigt und eingedampft. Das so erhaltene Produkt war mit kleinen Mengen Benzylmercaptan verunreinigt und wurde durch präparative Schichtchromatographie auf sechs 20 × 20 cm Silicagelplatten mit Hexan/Äthylacetat (4 : 1) weiter gereinigt. Das reine Produkt, 1-(Benzylthiomethyl-1-decarboxylasalocid, wurde als farbloser fester Schaum erhalten.

Beispiel 7

Eine Lösung von 3 g der gemäß Beispiel 2 erhaltenen Mannichbase, 3 ml Mercaptoessigsäure und 3 ml Triäthylamin in 100 ml Äthanol wurde 36 Stunden zum Rückfluß erhitzt. Das Äthanol wurde dann unter vermindertem Druck entfernt und der Rückstand in Methylenchlorid-Lösung mit verdünnter wäßriger Natriumcarbonatlösung, Wasser, 0,5 N Salzsäure und Wasser gewaschen. Nach Trocknen und Eindampfen wurde das Rohprodukt an Silicagel mit 4% Methanol in Chloroform chromatographiert. Da sich herausstellte, daß das Produkt Natriumionen aus anorganischem Material absorbierte, wurde das das Produkt enthaltende Eluat vor dem Eindampfen mit sehr verdünnter Salzsäure gewaschen. Das 1-[(Carboxymethyl)thiomethyl]-1-decarboxylasalocid wurde so als farbloser Schaum erhalten.

Beispiel 8

Eine Lösung von 1,85 g 1-(Diäthylaminomethyl)-1-decarboxylasalocid und 2 mm Triäthylamin in 50 mm Äthanol wurde 32 Stunden zum Rückfluß erhitzt, während ein langsamer Strom von Schwefelwasserstoff durch die Lösung geleitet wurde. Nach Abdampfen des Lösungsmittels unter vermindertem Druck wurde der Rückstand auf präparativen Schichtplatten (20 × 20 cm, Silicagel) chromatographiert. Das Produkt, 1-Mercaptomethyl-1-decarboxylasalocid, wurde als farbloser fester Schaum erhalten.

Beispiel 9

Eine Lösung von 1 g 1-(Diäthylaminomethyl)-1-decarboxylasalocid und 2 ml Triäthylamin in 50 ml Äthanol wurde 8 Stunden zum Rückfluß erhitzt, wobei gasförmiges Methylmercaptan eingeleitet wurde. Nach Eindampfen und Reinigung durch präparative Schichtchromatographie wurden 300 mg 1-(Methylthiomethyl)-1-decarboxylasalocid als farbloser fester Schaum erhalten.

Beispiel 10

Eine Lösung von 1,4 g 1-(Diäthylaminomethyl)-1-decarboxylasalocid, 2 ml Furfurylmercaptan und 2 ml Diäthylamin in 50 ml Äthanol wurden 42 Stunden zum Rückfluß erhitzt, wobei nach 18 Stunden je 2 ml zusätzliche Base und Mercaptan zugesetzt wurden. Nach Eindampfen und Reinigung durch präparative Schichtchromatographie wurde das Produkt, 1-(2-Furylmethylthiomethyl)-1-decarboxylasalocid, als farbloser fester Schaum erhalten.

Beispiel 11

Nach den oben beschriebenen Verfahren und unter Verwendung des passenden Thiols können Verbindungen der Formel I hergestellt werden, in denen $R^1$ Allyl, 2-Aminomethyl, 4-Aminomethyl, 4-Brom-3-methylphenyl, 4-Bromphenyl, 1-Butyl, 2-Butyl, tert.Butyl, Isobutyl, Carboäthoxymethyl, Carbomethoxymethyl, 2-Carboxyäthyl, o-Carboxyphenyl, 4-Chlorbenzyl, 2-n-Decylaminoäthyl, 2,5-Dichlorphenyl, 3,4-Dichlorphenyl, 2-Diäthylaminoäthyl, 2-Diisopropylaminoäthyl, 1-Dodecyl, Äthyl, 4-Fluorphenyl, n-Heptyl, n-Hexadecyl, 2-Hydroxyäthyl, 2-Äthoxyäthyl, 2-Äthylthioäthyl, 1-Methyl-2-imidazolyl, 2,3-Dihydroxypropyl, 2-Pyridyl, 4-Pyridyl, 3-Hydroxy-2-pyridyl, 2-Thiazolyl, 2-Methoxyphenyl, 3-Methoxyphenyl, 4-Methoxyphenyl, 2-Methyl-2-butyl, 3-Methyl-1-butyl, 4-Nitrophenyl, 1-Nonyl, 1-Octyl, Pentachlorphenyl, Pentafluorphenyl, 1-Pentyl, 3-Phenyl-1-propyl, 1-Propyl, 2-Propyl, 2-Chinolyl, 2,3,5,6-Tetrafluorphenyl, p-Tolyl, 2,4,5-Trichlorphenyl, 7-Trifluormethyl-4-chinolyl und Triphenylmethyl darstellt.

**Patentansprüche**

1. Verbindungen der Formel

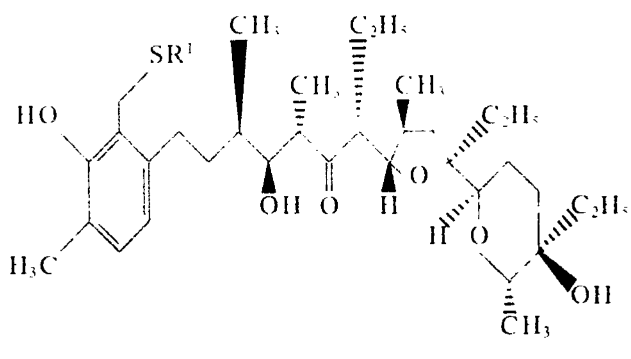

(I)

worin $R^1$ Phenyl, substituiertes Phenyl, Heteroaryl, Phenyl-$C_{1-7}$alkyl, $C_{4-7}$ Cycloalkyl, $C_{1-7}$ Alkyl, $C_{2-7}$ Alkenyl, Carbo-$C_{1-16}$alkoxy-$C_{1-7}$alkyl, Amino-$C_{1-16}$alkyl, $C_{1-7}$-Alkylamino-$C_{1-16}$alkyl, Di($C_{1-7}$alkyl)-amino-$C_{1-16}$alkyl, Hydroxy-$C_{1-16}$alkyl, $C_{1-16}$ Alkoxy-$C_{1-16}$alkyl, $C_{1-16}$-Thioalkoxy-$C_{1-16}$alkyl, Carboxy-$C_{1-7}$alkyl oder Wasserstoff bedeutet.

2. Verbindungen nach Anspruch 1, in denen $R^1$ Phenyl, substituiertes Phenyl, Phenyl-$C_{1-7}$alkyl, $C_{4-7}$ Cycloalkyl, $C_{1-7}$ Alkyl, Carboxy-$C_{1-7}$alkyl oder Wasserstoff ist.

3. 1-(Phenylthiomethyl)-1-decarboxylasalocid.

4. Verbindungen nach Anspruch 1, in denen $R^1$ Chlorphenyl, Cyclohexyl, Benzyl oder Carboxymethyl ist.

5. Pharmazeutische Präparate, gekennzeichnet durch einen Gehalt an einer Verbindung der Formel I gemäß Definition in Anspruch 1.

6. Verfahren zur Herstellung von Verbindungen der Formel I gemäß Definition in Anspruch 1, dadurch gekennzeichnet, daß man eine Verbindung der Formel

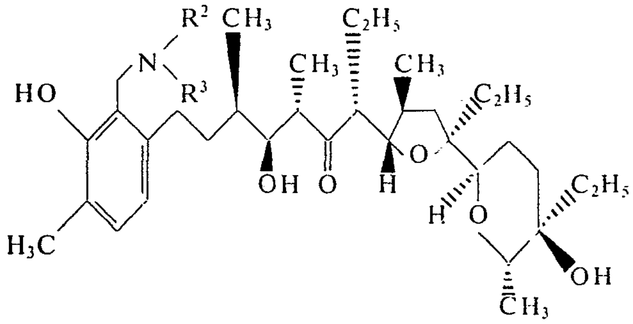

(II)

worin $R^2$ und $R^3$ $C_{1-7}$ Alkyl sind oder zusammen mit dem Stickstoffatom einen 5- oder 6gliedrigen heterocyclischen Ring, der ein weiteres Heteroatom enthalten kann, darstellen, mit einer Verbindung der Formel $R^1SH$, worin $R^1$ die in Anspruch 1 angegebene Bedeutung hat, in Gegenwart einer schwachen Base umsetzt.

7. Verfahren nach Anspruch 6, dadurch gekennzeichnet, daß $R^1$ Phenyl ist.

8. Verbindungen der Formel

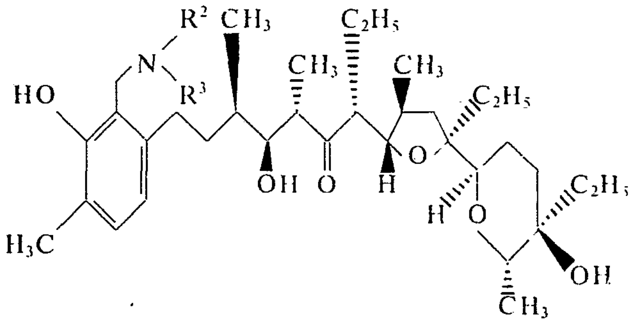

worin $R^2$ und $R^3$ $C_{1-7}$ Alkyl sind oder zusammen mit dem Stickstoffatom einen 5- oder 6gliedrigen heterocyclischen Ring, der ein weiteres Heteroatom enthalten kann, darstellen.

**Claims**

1. Compounds of the formula

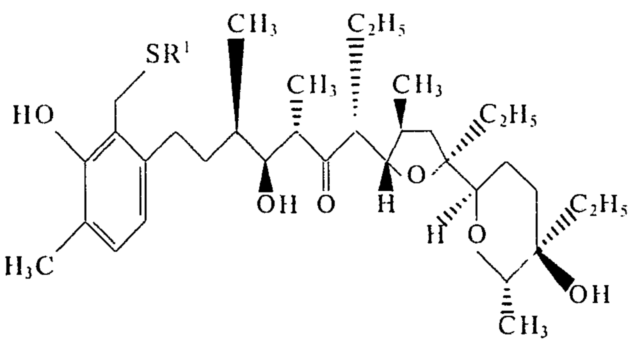

(I)

wherein $R^1$ signifies phenyl, substituted phenyl, heteroaryl, phenyl-$C_{1-7}$alkyl, $C_{4-7}$ cycloalkyl, $C_{1-7}$ alkyl, $C_{2-7}$ alkenyl, carbo-$C_{1-16}$alkoxy-$C_{1-7}$alkyl, amino-$C_{1-16}$ alkyl, $C_{1-7}$-alkylamino-$C_{1-16}$alkyl, di($C_{1-7}$alkyl)amino-$C_{1-16}$alkyl, hydroxy-$C_{1-16}$alkyl, $C_{1-16}$ alkoxy-$C_{1-16}$alkyl, $C_{1-16}$-thioalkoxy-$C_{1-16}$alkyl, carboxy-$C_{1-7}$alkyl or hydrogen.

2. Compounds according to claim 1, in which $R^1$ is phenyl, substituted phenyl, phenyl-$C_{1-7}$alkyl, $C_{4-7}$ cycloalkyl, $C_{1-7}$ alkyl, carboxy-$C_{1-7}$alkyl or hydrogen.

3. 1-(Phenylthiomethyl)-1-decarboxylasalocid.

4. Compounds according to claim 1, in which $R^1$ is chlorophenyl, cyclohexyl, benzyl or carboxymethyl.

5. Pharmaceutical preparations, characterised in that they contain a compound of formula I in accordance with the definition in claim 1.

6. Process for the manufacture of compounds of formula I in accordance with the definition in claim 1, characterised in that a compound of the formula

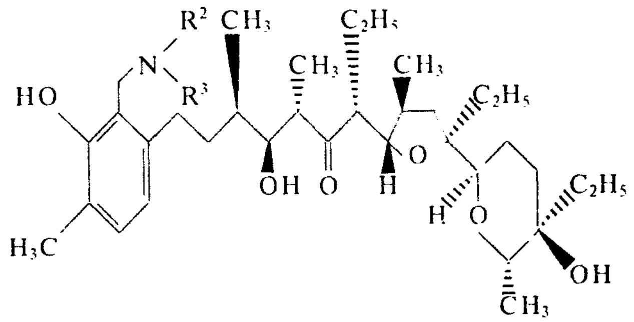

(II)

wherein $R^2$ and $R^3$ are $C_{1-7}$ alkyl or together with the nitrogen atom represent a 5- or 6-membered heterocyclic ring, which can contain a further hetero atom, is reacted with a compound of the formula $R^1SH$, wherein $R^1$ has the significance given in claim 1, in the presence of a weak base.

7. Process according to claim 6, characterised in that $R^1$ is phenyl.

8. Compounds of the formula

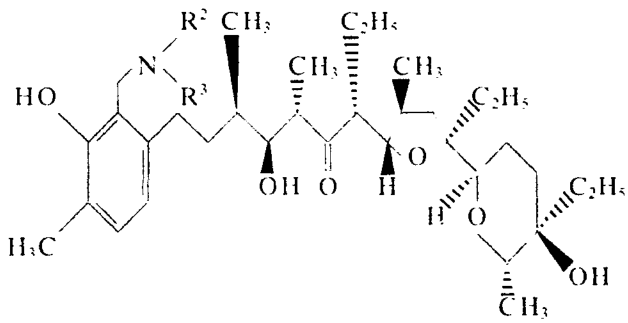

wherein $R^2$ and $R^3$ are $C_{1-7}$ alkyl or together with the nitrogen atom represent a 5- or 6-membered heterocyclic ring, which can contain a further hetero atom.

## Revendications

1. Composés de formule

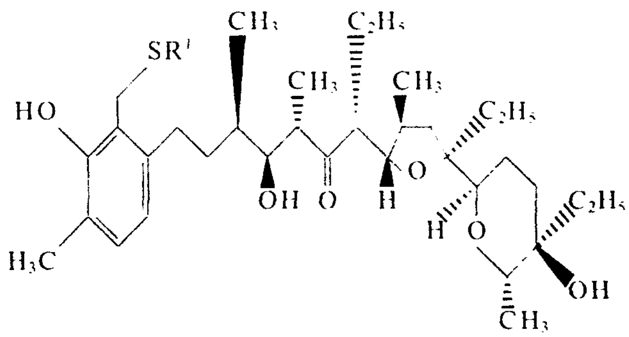

(I)

dans laquelle $R^1$ représente un groupe phényle, phényle substitué, hétéroaryle, phényl-(alkyle en $C_1-C_7$), cycloalkyle en $C_4-C_7$, alkyle en $C_1-C_7$, alcényle en $C_2-C_7$, carbo-(alcoxy en $C_1-C_{16}$)-(alkyle en $C_1-C_7$), amino-(alkyle en $C_1-C_{16}$), (alkyle en $C_1-C_7$)-amino-(alkyle en $C_1-C_{16}$), di-(alkyle en $C_1-C_7$)-amino-(alkyle en $C_1-C_{16}$), hydroxyalkyle en $C_1-C_{16}$, (alcoxy en $C_1-C_{16}$)-(alkyle en $C_1-C_{16}$), (thioalcoxy en $C_1-C_{16}$)-(alkyle en $C_1-C_{16}$), carboxy-(alkyle en $C_1-C_7$) ou l'hydrogène.

2. Composés selon la revendication 1, pour lesquels $R^1$ représente un groupe phényle, phényle substitué, phényl-(alkyle en $C_1-C_7$), cycloalkyle en $C_4-C_7$, alkyle en $C_1-C_7$, carboxy-(alkyle en $C_1-C_7$) ou l'hydrogène.

3. Le 1-(phénylthiométhyl)-1-décarboxylasalocide.

4. Composés selon la revendication 1, pour lesquels $R^1$ représente un groupe chlorophényle, cyclohexyle, benzyle ou carboxyméthyle.

5. Compositions pharmaceutiques caractérisées en ce qu'elles contiennent un composé de formule I selon la revendication 1.

6. Procédé de préparation des composés de formule I selon la revendication 1, caractérisé en ce que l'on fait réagir un composé de formule

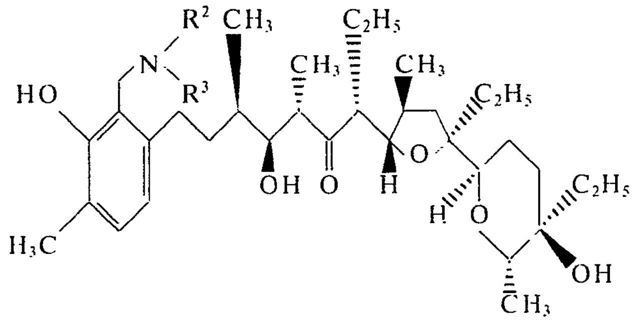

(II)

dans laquelle $R^2$ et $R^3$ représentent des groupes alkyles en $C_1-C_7$ ou forment ensemble et avec l'atome d'azote un noyau hétérocyclique à 5 ou 6 chaînons qui peut contenir un autre hétéroatome, avec un composé de formule $R^1SH$ dans laquelle $R^1$ a les significations indiquées dans la revendication 1, en présence d'une base faible.

7. Procédé selon la revendication 6, caractérisé en ce que $R^1$ est un groupe phényle.

8. Composés de formule

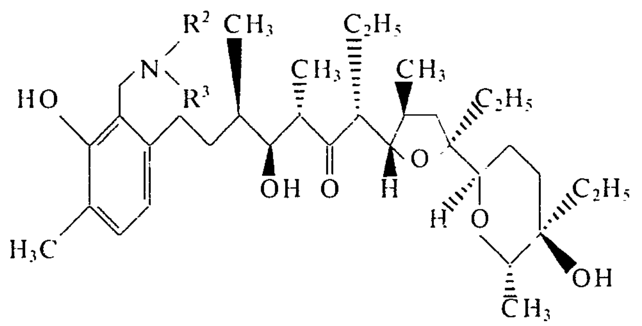

dans laquelle $R^2$ et $R^3$ représentent des groupes alkyles en $C_1-C_7$ ou forment ensemble et avec l'atome d'azote un noyau hétérocyclique à 5 ou 6 chaînons qui peut contenir un autre hétéroatome.